# FASCICULE DE BREVET EUROPEEN

(11) **EP 2 240 120 B1**
(45) Date de publication et mention de la délivrance du brevet: **04.07.2012**
(21) Numéro de dépôt: 08872036.2
(22) Date de dépôt: 02.12.2008
(51) Int. Cl.: A61F 2/20, A61M 16/04, A61L 27/14

(54) **DISPOSITIF À CLAPETS DESTINÉ À ÊTRE IMPLANTÉ AU SEIN D'UN LARYNX DYSFONCTIONNEL OU D'UNE PROTHÈSE DE LARYNX**
VENTILVORRICHTUNG ZU IMPLANTATION IN EINEN DYSFUNKTIONELLEN LARYNX ODER EINE LARYNXPROTHESE
VALVE DEVICE DESIGNED TO BE IMPLANTED IN A DYSFUNCTIONAL LARYNX OR IN A PROSTHETIC LARYNX

(30) Priorité: 03.12.2007 FR 0708427
(43) Date de publication de la demande: 20.10.2010
(73) Titulaire: PROTIP SAS, 67380 Lingolsheim (FR); Hôpitaux Universitaires de Strasbourg, 67091 Strasbourg Cedex (FR); Université Louis Pasteur, 67070 Strasbourg Cedex (FR)
(72) Inventeur: DEBRY, Christian, F-75015 Paris (FR); WALDER, André, F-75015 L'Hay-Les-Roses (FR); DUFFAIT, Roland, F-39700 Evans (FR); LEFEBRE, Sylvain, F-25840 Vuillafans (FR); BLANC, Claudia, D-98714 Stützerbach (DE)
(74) Mandataire: Barny, Luc
(86) Numéro de dépôt international: PCT/FR2008/001667
(87) Numéro de publication internationale: WO 2009/098408

(56) Documents cités:
- EP-A- 0 815 807
- WO-A-2004/060438
- DE-A1- 1 791 014
- DE-U1-202004 010 382
- FR-A- 2 559 067
- US-A- 5 765 560

## Description

La présente invention se rapporte au domaine des prothèses avec pour fonction la restauration à la fois de la déglutition, de la respiration et de la phonation chez un patient ayant subi, par exemple, une ablation totale ou partielle du larynx. Plus particulièrement, la présente invention concerne un système à clapets destiné à être disposé au sein d'une prothèse et dont la fonction consiste à permettre la respiration tout en assurant l'étanchéité vis-à-vis d'autres éléments comme la salive, le mucus ou tout autre élément issu du bol alimentaire.

Actuellement, en France plus de 3300 hommes et 500 femmes contractent annuellement un cancer du larynx. Les soins nécessitent dans certains cas, l'ablation totale du larynx et les patients perdent alors l'usage de la parole, la respiration et la formation de la voix ne pouvant pas se produire de manière naturelle. La respiration est alors classiquement assurée par une trachéotomie définitive.

Pour retrouver la capacité de parler, il existe différentes possibilités, comme la rééducation oesophagienne ou l'utilisation de prothèses phonatoires basées sur le principe des fistules trachéo-oesophagiennes. De tels dispositifs sont largement décrits dans l'art antérieur et donnent une certaine satisfaction tout en n'étant pas dénuées d'une certaine morbidité.

Il existe à ce jour de nombreux types de prothèses implantées au niveau de la paroi trachéo-oesophagienne. À titre d'exemples non limitatifs, on peut citer les inventions décrites dans les demandes de brevet US 4,911,716, US 4,614,516, US 4,435,853 ou encore US 5,391,205. Ces prothèses consistent en des tubes creux présentant pour la plupart, au niveau de leurs extrémités, des épaulements ou collerettes dont la fonction est de maintenir la prothèse en place dans la paroi trachéo-oesophagienne, et un clapet à charnière du côté oesophage, afin d'empêcher le passage des fluides de l'oesophage vers la trachée dans les voies respiratoires.

La demande de brevet EP 0 651 980 propose une prothèse dont l'invention repose sur la mise en place d'un second clapet anti-retour disposé dans le même sens et en série par rapport à un premier clapet.

Dans le même sens, il peut également être cité comme art antérieur le brevet EP 0 815 807 qui décrit également une prothèse destinée à remédier au dysfonctionnement du larynx, ladite prothèse consistant en un tube présentant au niveau de l'une de ses extrémités conformée en biseau d'une part une face d'obturation inclinée et, d'autre part, des orifices latéraux de communication entre l'intérieur et l'extérieur du tube formant la prothèse. En pratique, la face d'obturation inclinée dirige le bol alimentaire de la bouche vers l'oesophage alors que les orifices latéraux de communication permettent la circulation d'air dans les deux sens.

Il n'en reste pas moins vrai que par ceux-ci peuvent se produire des fuites et, plus particulièrement, des introductions de fluides ou de reflux de fluides ou de tout élément du bol alimentaire vers les voies respiratoires. En effet, dans la pratique, l'orientation de la face d'obturation n'est pas suffisante pour assurer à elle seule une totale étanchéité.

La présente invention vise à remédier à cet inconvénient en proposant un nouveau dispositif permettant d'une part d'assurer une parfaite étanchéité de la prothèse en empêchant toute fuite vers les voies respiratoires et, d'autre part, de permettre un écoulement de l'air dans les deux sens afin de permettre la respiration du patient ainsi que les fonctions de phonation et de déglutition. Un autre avantage de l'invention, comme il ressortira plus loin dans la description, réside dans le fait que le dispositif selon l'invention présente une excellente résistance à l'usure et a donc une meilleure durée de vie limitant ainsi les opérations de changement qui restent lourdes pour le patient.

Pour ce faire, la présente invention propose une toute nouvelle approche qui consiste à supprimer la présence même des orifices ou des ouvertures de communication. En effet, l'ensemble des dispositifs de l'art antérieur présente de tels orifices pour permettre le passage de l'air nécessaire à la respiration du patient. Cependant, ces orifices étant continuellement ouverts, des écoulements du bol ou de tout autre fluide finissent toujours par s'y écouler.

L'objet de l'invention est particulièrement innovant car il permet, tout en supprimant la présence d'orifices de communication, d'assurer le passage de l'air à l'inspiration et à l'expiration, et ainsi de rétablir les fonctions de déglutition, respiration et phonation.

Plus particulièrement, la présente invention concerne, de manière générale, un dispositif à clapets destiné à être implanté soit au sein d'un larynx dysfonctionnel, soit couplée à une prothèse artificielle de larynx, ledit dispositif comportant une portion distale formant armature porteuse annulaire et une portion centrale formant obturateur destiné à permettre le passage de l'air et à empêcher de manière hermétique le passage de tout autre élément, ledit dispositif étant caractérisé en ce que l'obturateur comprend i) une partie périphérique formant un premier clapet solidaire de l'armature porteuse annulaire au niveau d'une première région charnière, ledit premier clapet étant capable de s'abaisser sous l'effet de la dépression résultant de l'inspiration du patient et ii) une partie centrale formant un second clapet solidaire du premier clapet au niveau d'une seconde région charnière, ledit second clapet étant capable non seulement de se soulever sous l'effet de la surpression exercée par l'air expiré par le patient mais également de coopérer avec le premier clapet pour s'abaisser suite à l'inspiration du patient, lesdits premier et second clapets coopérant entre eux de manière totalement hermétique.

Dans la présente description, les expressions « clapet », « valve », « obturateur » ou encore « soupape » peuvent être utilisées de manière interchangeable pour désigner un élément mobile capable de passer d'une position fermée, ou d'obturation, à une position ouverte permettant le passage de l'air.

Les expressions « distales » et « centrales » ont pour référentiel le centre du dispositif selon l'invention. Il s'en suit que l'expression « portion distale » fait référence à la portion la plus éloignée du centre du dispositif par opposition à l'expression « portion centrale » qui fait référence à la portion la plus proche du centre dudit dispositif selon l'invention.

Selon une forme de réalisation préférée, la structure circulaire des deux clapets est préférable du fait que, pour un encombrement donné c'est cette conformation qui offre la plus grande ouverture pour le passage de l'air Cependant, une telle forme n'est aucunement limitative et le dispositif selon l'invention peut présenter d'autres formes ou structures notamment de section ovoïde.

Une autre formulation consiste à dire que l'invention repose sur la coopération de deux clapets imbriqués l'un dans l'autre dans le même plan au repos, l'ensemble des deux clapets s'ouvrant à l'inspiration alors que seul le plus petit s'ouvre à l'expiration.

Le fonctionnement des clapets est réalisé par la surpression et la dépression dans le poumon pendant les phases d'expiration et d'inspiration. À l'inspiration du fait de la dépression existant dans les poumons les deux clapets s'abaissent dans le même mouvement. A l'expiration, du fait de la surpression existant dans les poumons seul le petit clapet s'ouvre vers l'extérieur du dispositif, le grand clapet restant bloqué sur son siège. La garantie d'une bonne étanchéité de la trachée à la fermeture des clapets, objet de l'invention, est un élément très important pendant la déglutition pour éviter aux aliments ou à la salive de pénétrer dans la trachée puis dans les poumons.

Comme il ressortira des exemples ci-dessous, une des caractéristiques de l'invention repose sur l'épaisseur même des clapets, cette dernière déterminant, avec la nature du matériaux choisi, l'élasticité du clapet au niveau des régions charnières et donc influant directement sur sa capacité à s'abaisser ou bien à maintenir sa position. Cette élasticité doit être calculée de manière à pouvoir supporter la pression correspondant à la poussée exercée par la passage des aliments dans les voies aériennes digestives ou à une accumulation de salive ou mucus tout en étant capable de s'abaisser ou de se soulever suite à une surpression expiratoire ou à une dépression inspiratoire.

Dans la suite de la description, l'expression « bol alimentaire » sera utilisée pour définir non seulement tout élément dudit bol alimentaire, mais également le mucus, la salive ou tout autre élément ou corps étranger aux voies respiratoires.

Selon une forme de réalisation préférée de l'invention, le dispositif est caractérisé en ce que l'armature porteuse annulaire comprend au moins un élément de butée empêchant le soulèvement du premier clapet.

Cet élément de butée joue un rôle clé car il permet d'empêcher tout mouvement du premier clapet vers le haut suite à l'expiration du patient.

Ledit élément de butée peut consister en tout moyen venant entraver et bloquer le mouvement du premier clapet vers le haut, comme par exemple un ergot faisant saillie sur l'ensemble de la circonférence interne de l'armature porteuse annulaire ou bien simplement disposé en un ou plusieurs points précis de cette circonférence.

Selon une forme d'exécution préférée, il est envisagé que ledit élément de butée consiste en ce que la circonférence externe du premier clapet de l'obturateur soit découpée en forme de biseau « vers le bas » et en ce que la circonférence interne de l'armature porteuse annulaire se trouvant en vis-à-vis soit également découpée en biseau « inversé vers le haut », les deux découpes en biseau coopérant entre elles de manière à bloquer le soulèvement du premier clapet.

La forme de biseau est préférée car elle permet d'assurer une meilleure étanchéité mais toute autre forme permettant une telle coopération doit bien évidemment être considérée comme équivalente.

Cette découpe présente, outre l'avantage de sa simplicité, d'éviter l'adjonction d'une pièce supplémentaire comme d'un ergot, ce qui permet de limiter les coûts de fabrication et surtout d'éviter la présence d'une pièce faisant saillie et donc susceptible de s'user et, par la suite, de se rompre entraînant alors la nécessité d'une intervention pour pouvoir remédier à cette rupture.

Comme il a été décrit plus haut, si le premier clapet n'est capable que de s'abaisser sous l'effet de la dépression exercée sur sa face inférieure du fait de l'inspiration du patient, le second clapet peut, quant à lui, non seulement s'abaisser de la même manière mais également se soulever sous l'effet, cette fois-ci, de la surpression exercée au niveau de sa face inférieure suite à l'expiration du patient. On comprendra aisément que le premier clapet subit la même surpression mais que, du fait de la présence d'un élément de butée, ce dernier ne peut aucunement se soulever.

En ce qui concerne l'abaissement du second clapet, celui-ci est de préférence simultané à l'abaissement du premier clapet. Selon une forme préférée de l'invention, il est prévu un moyen de coopération visant à assurer cette synchronisation. Plus particulièrement, les premier et second clapets comprennent un moyen de coopération de manière à permettre le soulèvement uniquement du second clapet et l'abaissement simultané des premier et second clapets.

Pour les mêmes raisons que celles énumérées plus haut au regard de l'élément de butée, la présente invention préfère un moyen de coopération qui consiste en ce que la circonférence interne du premier clapet soit découpée en biseau « vers le bas » et en ce que la circonférence externe du second clapet soit découpée en biseau « inversé vers le haut », les deux découpes en biseau coopérant entre elles.

Bien évidemment, ici aussi, tout moyen ou élément équivalent doit être considéré comme compris dans l'étendue de la protection conférée par la présente demande de brevet. Cependant, le principe des découpes en biseau, comme vu plus haut, est le seul à assurer une étanchéité parfaite entre les deux clapets.

Comme il ressort de la présente description, les deux clapets ne sont actionnés que par les effets de dépression et de surpression découlant de la respiration du patient et, à contrario, doivent être immobiles suite à une pression autre comme le poids de tout élément étranger pouvant provenir, par exemple, du bol alimentaire.

Les inventeurs, après de multiples essais, ont pu déterminer une épaisseur de matériau pour les clapets, ainsi que les régions charnières desdits clapets, qui permette de répondre à ces critères.

Cette caractéristique est tout à fait nouvelle en ce sens que, pour l'ensemble des dispositifs existants et présentant une valve anti-retour, cette valve ne pouvait que se soulever suite à l'expiration du patient et cette dernière venait en appui sur l'extrémité de la prothèse afin de na pas s'affaisser sous le poids du bol alimentaire (les échanges d'air s'effectuant, comme décrit plus haut, par des ouvertures de communication).

Pour la première fois, il a été calculé une épaisseur de matériau permettant, à elle seule, d'assurer une rigidité suffisante pour pouvoir résister au poids du bol alimentaire sans s'abaisser tout en assurant une élasticité suffisante pour pouvoir s'abaisser et/ou se soulever sous les différentes pressions d'air liées à la respiration.

En effet, comme il ressortira plus en détail des exemples ci-dessous, le poids du bol alimentaire ne dépasse pas, en moyenne, 7 g, ce qui se traduit par une pression n'excédant pas, en moyenne, 0, 3. 10⁻³ MPa ou N/mm². Le bol alimentaire est constitué d'aliments partiellement ou totalement mâchés et imprégnés de salive. Suite à la déglutition, ce bol alimentaire va parcourir l'oesophage pour atteindre l'estomac. Sa masse va être diffuse suite à la mastication et la déglutition et suivant la nature et le poids des aliments qui le constituent, son trajet dans l'oesophage va être plus ou moins rapide. En conditions physiologiques, on peut donc raisonnablement considérer que la totalité dudit bol alimentaire ne viendra pas atteindre un point précis du dispositif selon l'invention au même instant. Par conséquent, la pression de 0,3.10⁻³ MPa ou N/mm² évoquée ci-dessus est considérée comme la pression maximale pouvant être exercée sur le dispositif. Le dispositif à clapets selon l'invention est par conséquent conçu pour résister à une pression inférieure à celle nécessaire pour supporter un bol alimentaire de 7g et ceci est amplement suffisant pour un bon fonctionnement du dispositif à clapets.

Par contre, la surpression exercée lors de l'expiration du patient et la dépression exercée lors de l'inspiration, étant de l'ordre de 10⁻² MPa, c' est-à-dire que toutes deux sont largement supérieures à la pression exercée par le bol alimentaire, il est aisé de comprendre que les clapets pourront se soulever ou s'abaisser lors de la respiration du patient.

Plus particulièrement, le dispositif selon l'invention est caractérisé en ce que les premiers clapets et la première charnière sont dimensionnés de sorte à pouvoir supporter une charge comprise entre 0 et 6 g sans se soulever et/ou s'abaisser, selon le mode de réalisation de l'invention choisi.

Dans la réalisation du dispositif selon l'invention, cela se traduit par le fait que lesdites première et seconde régions charnières présentent une épaisseur comprise entre 0,5 et 3,5 mm, préférentiellement de moins de 2,5 mm et encore plus préférentiellement de 1 mm.

Dans un autre mode de réalisation de l'invention, cela se traduit par le fait que lesdits premier et second clapets présentent une épaisseur comprise entre 0,5 et 3,5 mm, préférentiellement de moins de 2,5 mm et encore plus préférentiellement de 1 mm.

Selon une variante, il peut être souhaité que les résistances des deux clapets soient différentes afin de pouvoir s'adapter, par exemple, à des patients présentant des problèmes respiratoires entraînant des différences de pressions entre l'inspiration et l'expiration. Dans ce cas précis, les dimensions des deux clapets, ainsi que des deux régions charnières correspondantes, peuvent être différentes.

Cependant, de manière générale, il est préféré que lesdits premier et second clapets ainsi que lesdites première et seconde régions charnières présentent la même épaisseur.

Cette dernière forme d'exécution présente de nombreux avantages comme, par exemple, la réduction des coûts de fabrication.

Dans un mode de réalisation préféré selon l'invention, afin d'augmenter la résistance à la pression exercée par les fluides tout en n'empêchant pas le fonctionnement des clapets, un dispositif d'assistance pouvant être mécanique, électrique ou électronique est utilisé. Ledit dispositif d'assistance est placé au niveau de l'armature porteuse annulaire et/ou au niveau du premier clapet. La présente invention porte également sur un dispositif à clapets pour prothèses de larynx dont l'armature porteuse annulaire et/ou le premier clapet sont munis d'un dispositif d'assistance pouvant être mécanique, électrique ou électronique.

Dans un mode de réalisation particulier, le dispositif d'assistance décrit ci-dessus est un dispositif aimanté. Par dispositif aimanté ou élément aimanté, on entend un ou plusieurs aimants permanents de type lanthanides qui soient biocompatibles ou rendus biocompatibles par différents traitements connus de l'homme de l'art. Préférentiellement, ce ou ces aimants sont choisis parmi les aimants permanents de type lanthanides dont la rémanence est comprise entre 1080 et 1150 mT. Ledit dispositif aimanté peut être placé soit sur le premier clapet, soit sur la surface interne de l'armature porteuse du dispositif selon l'invention. En vis-à-vis du ou des aimants quand le premier clapet est en position fermée, on place un élément métallique. Par élément métallique, on entend un ou plusieurs éléments métalliques susceptibles d'être aimantés qui sont placés de façon à entrer en contact avec le ou les aimants quand le premier clapet est en position fermée. La nature du dispositif aimanté pourra être adaptée à chaque situation en faisant varier le nombre d'aimants et/ou leur position par exemple.

Dans un mode de réalisation particulier, la présente invention porte sur un dispositif à clapets pour prothèse de larynx comportant un dispositif d'assistance qui consiste en un élément métallique disposé au niveau de l'armature porteuse annulaire venant en contact avec un élément aimanté disposé au niveau du premier clapet.

Dans un mode de réalisation préféré, la présente invention porte sur un dispositif à clapets pour prothèse de larynx comportant un dispositif d'assistance qui consiste en un élément métallique disposé au niveau du premier clapet venant en contact avec un élément aimanté disposé au niveau de l'armature porteuse annulaire.

Le dispositif aimanté décrit ci-dessus a une fonction d'assistance mécanique du dispositif à clapets selon l'invention. De manière plus générale, il sera facile pour l'homme du métier de remplacer ou de compléter ce dispositif aimanté par tout dispositif assurant une fonction équivalente ou susceptible d'améliorer cette fonction, tel qu'un autre dispositif d'assistance pouvant être mécanique, électrique ou électronique.

En outre, les régions charnières et les clapets peuvent ne pas présenter les mêmes épaisseurs, ces dernières pouvant être adaptées à une situation précise dictée par l'état même du patient.

Il découle de l'invention même que, pour pouvoir assurer l'élasticité nécessaire au bon fonctionnement des clapets, au moins les régions charnières doivent être en un matériau semi-rigide. Par « semi-rigide », il faut comprendre un matériau suffisamment souple pour pouvoir assurer une certaine élasticité tout en étant assez rigide pour pouvoir résister et rester en forme sous l'effet d'une faible pression. Dans un mode de réalisation, seules les régions charnières peuvent être en un matériau semi-rigide, les clapets eux-mêmes pouvant être en matériau rigide.

Cependant, un mode préféré de l'invention est celui où non seulement les régions charnières mais aussi les clapets sont réalisés en un matériau semi-rigide pouvant être identique ou différent.

Selon une forme d'exécution encore plus préférée, le dispositif selon l'invention est caractérisé en ce qu'il est constitué intégralement d'un matériau semi-rigide.

Tout matériau semi-rigide, tel que défini plus haut, pourra être utilisé. Cependant, il peut être cité à titre d'exemple non limitatif le plastique, la gomme, une résine ou encore le silicone. Un matériau préféré consiste en le silicone 65 Shore A.

Selon une forme de réalisation préférée, le dispositif selon l'invention est caractérisé en ce que ledit matériau semi-rigide consiste en le silicone.

Selon encore un mode de réalisation préféré, l'ensemble du dispositif est réalisé en un matériau semi-rigide préférentiellement en silicone. Ce matériau présente l'avantage d'être biocompatible et permet d'éviter, ou du moins de limiter, par exemple tout rejet par le patient.

Cependant, afin de faciliter le maintien du dispositif, il peut être souhaité que celui-ci présente une certaine rigidité au niveau de la zone assurant son maintien, c'est-à-dire au niveau de l'armature porteuse annulaire.

Plus particulièrement, l'armature porteuse annulaire est renforcée par une ossature en titane, préférentiellement en titane poreux.

Le titane, présente l'avantage d'être biocompatible.

Un autre avantage de la présente invention est que ledit dispositif peut être adapté à tout type de prothèse de larynx existante.

Pour ce faire, l'armature porteuse annulaire présente, au niveau de sa circonférence externe, un moyen formant collerette de fixation amovible sur la prothèse de larynx.

De la sorte, le dispositif selon l'invention peut être monté de manière amovible sur une prothèse de larynx déjà installée.

Tout moyen de fixation amovible peut être utilisé mais il est cependant préféré un système à baïonnette, un système en anneaux enclipsables ou encore en un système à vis.

Bien évidemment, tout moyen équivalent doit être considéré comme faisant partie de l'invention.

Enfin, selon un dernier aspect de l'invention, il est également visé une prothèse de larynx comprenant, monté amovible comme vu plus haut ou directement intégré en son sein, un dispositif selon l'invention. La présente demande décrit l'utilisation d'un dispositif selon l'invention au sein d'une prothèse artificielle de larynx. Plus particulièrement, ledit dispositif est positionné au niveau de l'extrémité supérieure de ladite prothèse. L'invention concerne une prothèse artificielle de larynx comprenant, au niveau de sa portion supérieure, un dispositif selon l'invention tel que décrit plus haut.

Selon une première forme d'exécution, la prothèse artificielle est caractérisée en ce que ledit dispositif selon l'invention est disposé perpendiculairement à l'axe longitudinal de la prothèse.

Selon une seconde forme d'exécution, dont la réalisation ressortira clairement des exemples ci-après, le dispositif selon l'invention est disposé incliné par rapport à l'axe longitudinal de la prothèse selon un angle compris entre 0 et 60 degrés.

L'invention sera mieux comprise à la lumière des exemples ci-après ainsi que des figures suivantes, dans lesquelles :
La figure 1 représente, vu en coupe, un premier mode de réalisation d'un dispositif selon l'invention,
La figure 2 représente, vu en coupe, un second mode de réalisation d'un dispositif selon l'invention,
La figure 3 représente le dispositif selon l'invention de la figure 2 en phase d'expiration du patient,
La figure 4 représente le dispositif selon l'invention de la figure 2 en phase d'inspiration du patient,
La figure 5 représente une vue du dessus d'un dispositif selon l'invention,
La figure 6 représente le schéma du montage pour la mesure de la déformée en fonction de la pression,
La figure 7 représente, sous forme graphique, les résultats obtenus pour la mesure de la déformée,
La figure 8 représente le schéma de montage pour la mesure du débit d'air,
La figure 9 représente, sous forme graphique, les résultats obtenus pour la mesure du débit d'air,
La figure 10 représente un schéma du montage réalisé pour la mesure du test de fatigue.

### EXEMPLES

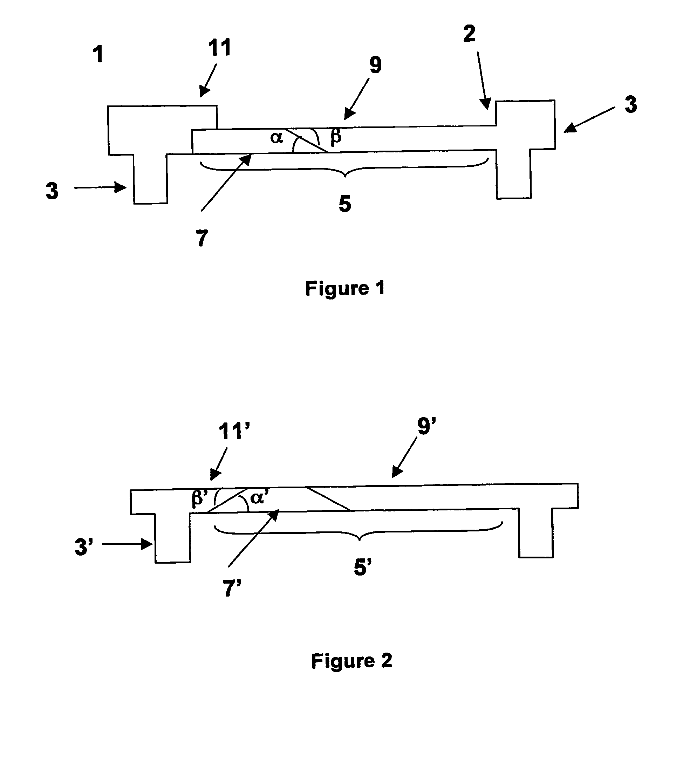

Si l'on se reporte à la figure 1, il est représenté un dispositif 1 selon l'invention en coupe longitudinale. Le dispositif comprend une armature porteuse annulaire 3 correspondant à la circonférence externe la plus grande du dispositif 1. Telle que représentée sur cette figure, ladite armature porteuse présente, en coupe, une forme en T, la barre verticale du T venant s'insérer au sein de la prothèse alors que l'extrémité externe de la barre horizontale vient prendre appui sur l'extrémité supérieure de la prothèse empêchant ainsi le dispositif 1 de bouger et de s'enfoncer au sein de la prothèse de larynx. Bien évidemment, une telle forme en T n'est aucunement limitative et toute forme assurant la même fonction doit être considérée comme équivalente.

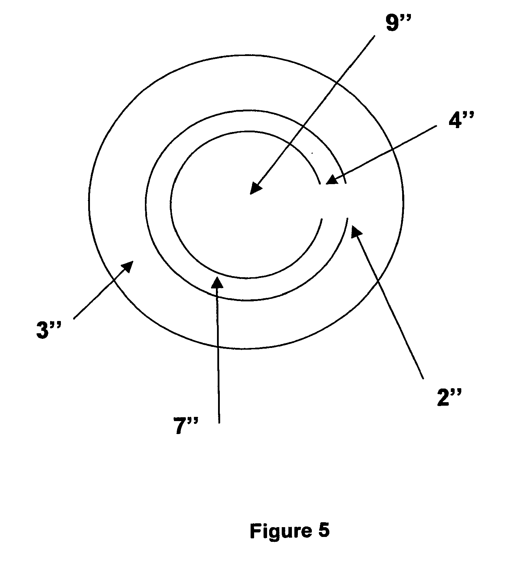

Sur cette même figure 1, la portion centrale formant obturateur 5 est bien visible au centre et comprend un premier clapet 7 et un second clapet 9, lesdits premier et second clapets 7 et 9 étant solidaires de l'armature porteuse annulaire 3 au niveau respectivement des régions charnières 2 et 4 (sur cette figure, pour un soucis de clarté, seule la région charnière 2 a pu être représentée, les deux régions charnières apparaissant clairement sur la figure 5). Cette figure étant une coupe faite au niveau des régions charnières 2 et 4, il faut comprendre que le premier clapet 7 fait le tour du second clapet 9 à l'exception des régions charnières 2 et 4. Ce dernier point ressortira plus clairement de la figure 5 qui représente le dispositif 1 vu de dessus. Sur cette figure 1 est montré un élément de butée 11 sous la forme d'un ergot faisant saillie de l'armature porteuse annulaire 3 vers l'intérieur de manière à venir bloquer tout mouvement du premier clapet 7 vers le haut. Cet élément 11, représenté ici également en coupe peut être présent sur l'ensemble de la circonférence interne de l'armature porteuse annulaire 3 ou bien seulement ponctuellement.

Cette figure 1 met également clairement en évidence la nature du moyen de coopération entre le premier clapet 7 et le second clapet 9. Plus particulièrement, la circonférence interne ou centrale du premier clapet 7 est découpée en forme de biseau « vers le bas », c'est-à-dire que la surface supérieure dudit premier clapet 7 est plus courte que la surface inférieure résultant ainsi en un biseau formant un angle α strictement compris entre 90 et 180 °. À l'opposé, la circonférence externe ou distale du second clapet 9 est découpée également en biseau, mais en biseau « inversé vers le haut », c'est-à-dire que la surface supérieure dudit second clapet 9 est, cette fois-ci, plus longue que la surface inférieure aboutissant ainsi à une découpe en biseau d'un angle β strictement compris entre 0 et 90°. Bien entendu, pour une bonne coopération des premier et second clapets 7 et 9, la somme des deux angles α et β doit être égale à 180°, ce qui revient à dire que lesdits deux angles α et β sont complémentaires.

La figure 2 représente une autre forme d'exécution de l'invention dans laquelle l'élément de butée 11' faisant saillie de l'armature porteuse annulaire 3' est remplacé par une découpe particulière en biseau. Plus particulièrement, la circonférence externe ou distale du premier clapet 7' est découpée en biseau « vers le bas », c'est-à-dire que la surface supérieure de ladite circonférence externe ou distale est plus courte que la surface inférieure de manière à former un biseau dont l'angle α' est strictement compris entre 90 et 180°. A l'opposé, la circonférence interne ou centrale de l'armature porteuse annulaire 3' est, quant à elle, découpée en biseau « inversé vers le haut », c'est-à-dire que la surface supérieure de cette circonférence interne ou centrale est plus longue que la surface inférieure de manière à former également un biseau dont l'angle β' est, dans ce cas, compris strictement entre 0 et 90°. De manière similaire au moyen de coopération entre les premier et second clapets 7 et 9 décrit plus haut, la somme des angles α' et β' doit être égale à 180°.

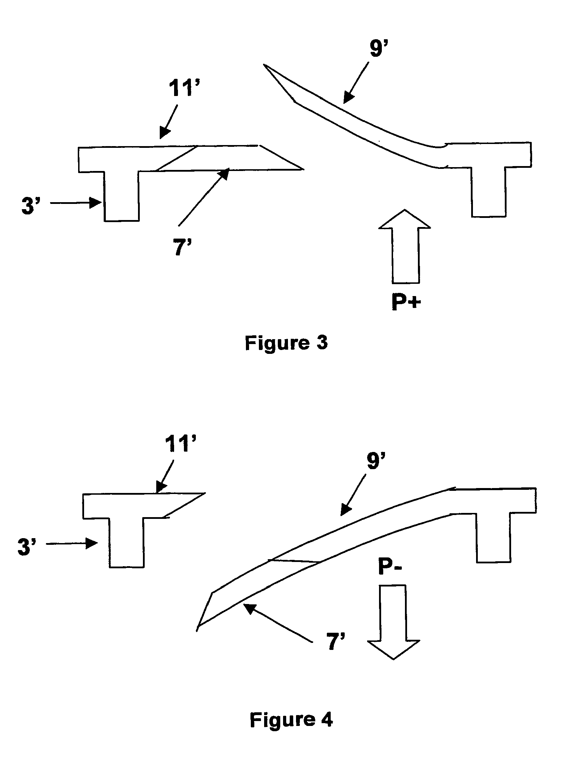

La figure 3 représente le dispositif selon l'invention décrit plus haut lors de l'expiration du patient. Sur cette figure, il ressort bien que seul le second clapet 9' est capable de se soulever sous l'effet de la surpression P⁺ exercée par le rejet d'air du patient lors de l'expiration, surpression qui vient pousser l'ensemble des deux clapets 7' et 9', ledit premier clapet 7' étant quant à lui incapable de se soulever du fait de la découpe en biseau formant élément de butée 11'.

La figure 4 représente le même dispositif lors de la phase d'inspiration au cours de laquelle une dépression P⁻ se crée au niveau des surfaces inférieures respectivement des premier et second clapets 7' et 9', cette dépression entraînant alors l'abaissement desdits premier et second clapets 7' et 9', le premier clapet 7' s'abaissant simultanément avec le second clapet 9' du fait de la découpe en biseau formant moyen de coopération.

Comme décrit plus haut, la figure 5 représente une vue de dessus du dispositif selon l'invention. Il ressort de cette figure que l'ensemble du dispositif, c'est-à-dire l'armature porteuse annulaire 3", le premier clapet 7" ainsi que le second clapet 9" présentent une forme circulaire. Une telle forme circulaire n'est aucunement limitative et il peut être envisagé d'autres formes comme des formes ovales, carrées, en étoile, etc.

Cette figure montre l'armature porteuse annulaire 3" puis, formant un cercle plus petit le premier clapet 7" et, formant un cercle encore plus petit, le second clapet 9". Le premier clapet 7" est solidaire de l'armature porteuse annulaire 3" au niveau d'une région charnière 2" alors que le second clapet 9" est solidaire, quant à lui, du premier clapet au niveau de la région charnière 4". La région charnière 4" se trouvant dans le prolongement de la région charnière 2", il apparaît donc que le second clapet 9", comme le premier clapet 7", sont tous deux solidaires, directement ou indirectement, de l'armature porteuse annulaire 3".

Les exemples ci-dessous permettent d'illustrer certaines caractéristiques de l'invention qui ont été mises en évidence par les inventeurs.

### Exemple 1 : MESURE DE DÉFORMATION ET DE DÉBIT D'AIR

### a. Mesure de déformation

Dans cette partie, la déformée de la valve en silicone pour différentes valeurs de pression exercée sur celle-ci a été mesurée.

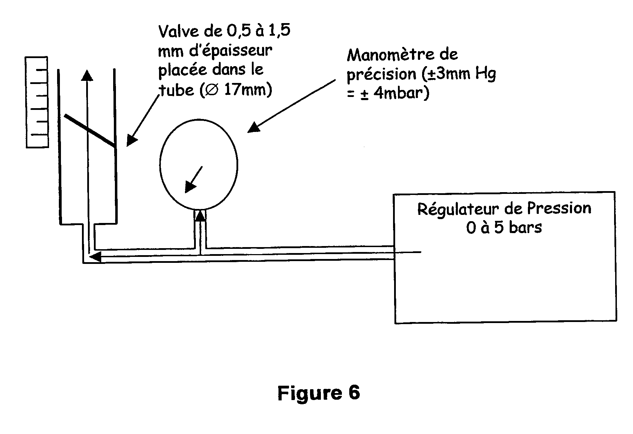

Le schéma du montage pour la mesure de la déformée en fonction de la pression est représenté à la figure 6.

Les résultats obtenus sont regroupés dans le tableau 1 ci-dessous.

**Tableau 1**

| **Pression (bar)** | **Pression (mmHg)** | **Valve 0,5 mm MED 4750 (mm)** | **Valve 0,5 mm MED 4735 (mm)** | **Valve 0, 7mm MED 4750 (mm)** | **Valve 1 mm MED 4750 (mm)** | **Valve 1 mm MED 4765 (mm)** | **Valve 1,5mm MED 4750 (mm)** |
|---|---|---|---|---|---|---|---|
| **0,03** | **22,5** | 7 | 7,5 | 6,75 | 5,5 | 5,75 | 4,5 |
| **0,04** | **30** | 7,5 | 7,5 | 7 | 5,75 | 5,75 | 5 |
| **0,05** | **37,5** | 8,5 | 8,5 | 7,75 | 6,25 | 6,25 | 5,5 |
| **0,06** | **45** | 8,75 | 8,5 | 8,25 | 6,5 | 6,5 | 5,75 |
| **0,07** | **52,5** | 9,5 | 9,5 | 9 | 6,5 | 6,5 | 6 |
| **0,08** | **60** | 10,5 | 10,5 | 9,25 | 6,75 | 6,75 | 6,25 |
| **0,09** | **67,5** | 11 | 11 | 9,5 | 7 | 7 | 6,5 |
| **0,1** | **75** | 11 | 11 | 9,5 | 7 | 7 | 6,75 |

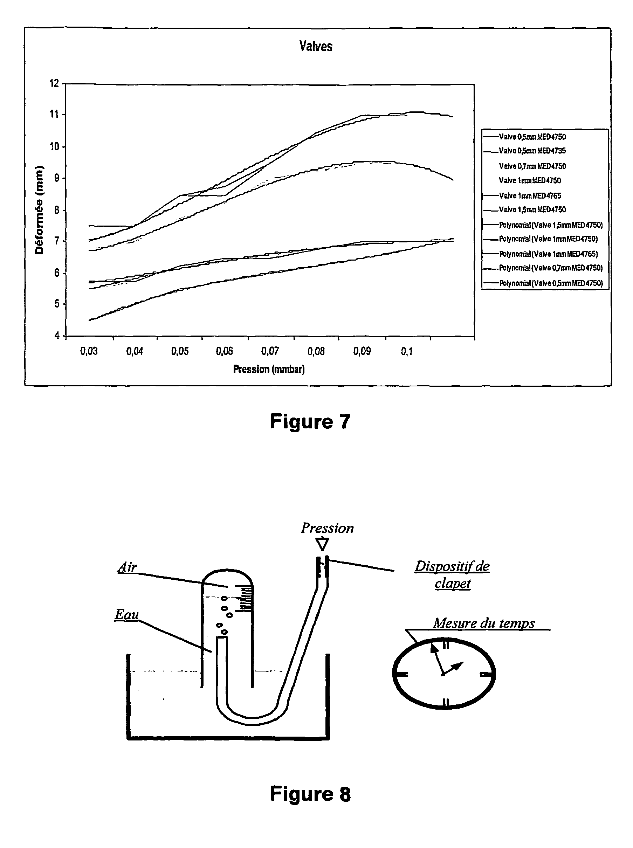

La représentation des résultats sous forme graphique est montrée à la figure 7.

### Pour la valve de 0,5 mm :

- Lors de la mise sous pression, on observe une déformation rapide de la valve, ce qui conduit à un passage d'air relativement important.
- Lorsque l'on inspire et que l'on expire à l'intérieur du tube, on ne relève aucun gène.
- Quelques frottements sont relevés au niveau de la valve et du tube, à haute pression (> 0,3 bar), provoquant une déformation des côtés de la valve.

### Pour la valve de 0, 7 mm :

- La déformée de la valve est un peu plus petite que pour la valve de 0,5. Le passage de l'air est toujours aussi bien assuré.
- Lors d'une série d'inspiration et d'expiration on ne relève aucun gène.
- Les mêmes frottements que pour la valve de 0,5 sont relevés.

### Pour la valve de 1 mm :

- On relève une déformée plus petite par rapport aux précédentes valves tout en assurant un passage d'air correct.
- La respiration se fait sans effort à fournir.
- Les mêmes frottements que pour la valve de 0,7 sont relevés.

### Pour la valve de 1, 5 mm :

- Idem que pour la valve précédente.

A partir des courbes, on notera un effet de saturation dû notamment aux frictions des cotés de la valve sur les parois du tube. Cet effet et relativement amoindri pour les valves de 1 mm et 1,5 mm car celles-ci se déforment moins, cependant la valve de 1 mm reste la plus appropriée. La dureté n'a pratiquement aucun effet notoire sur les mesures.

### b. Mesure de débit d'air

Le débit a été mesuré par le volume d'air stocké pendant une durée donnée dans un tube à essais gradué rempli d'eau, retourné sur une cuve contenant également de l'eau.

Le schéma du montage est représenté à la figure 8.

Les résultats obtenus sont représentés dans le tableau 2 ci-dessous.

**Tableau 2**

| **Pression (bar)** | **Pression (mmHg)** | **Valve 0,5 mm MED 4750 (cm3/s)** | **Valve 0,7mm MED 4750 (cm3/s)** | **Valve 1mm MED 4765 (cm3/s)** | **Valve 1,5mm MED 4750 (cm3/s)** |
|---|---|---|---|---|---|
| **0,03** | **22,5** | 25 | 20 | 15,43 | 14,88 |
| **0,04** | **30** | 68 | 52,08 | 47,16 | 44,64 |
| **0,05** | **37,5** | 92,3 | 79,42 | 63,2 | 56,8 |
| **0,06** | **45** | 113,1 | 92,6 | 76,52 | 65,7 |
| **0,07** | **52,5** | 127,2 | 108,8 | 86,9 | 77,9 |

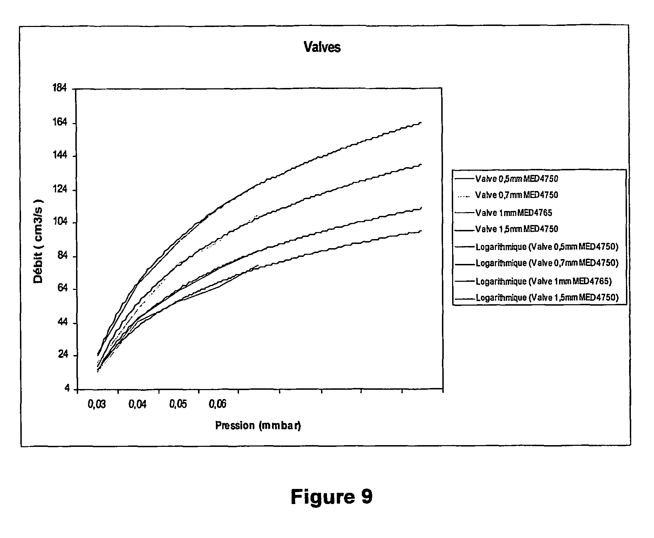

Les résultats sous forme graphique sont également représentés à la figure 9.

On remarque une très légère saturation de la valeur du débit pour des valeurs élevées de pression d'air, ce qui correspond à la butée de la membrane sur la paroi, remarquée précédemment dans la mesure de la déformée.

### Exemple 2 : TEST DE FATIGUE

Ces tests de fatigue ont pour but d'étudier le comportement et les déformations de la valve après deux millions de cycle de respiration, ce qui correspond à un fonctionnement de trois mois.

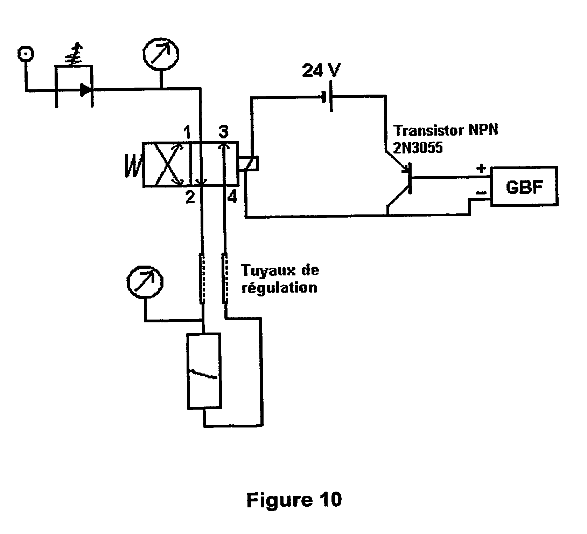

Le schéma du dispositif est représenté à la figure 10.

Les essais ont été poursuivis jusqu'à 20 millions de cycles (plus de 900 jours de fonctionnement réel), avec pli dans les deux sens sous une pression alternée de 22 mm de Hg, soit 30 mbar, à une fréquence de 10 Hz ; aucune déformation de la membrane n'est apparue ; seule la zone de charnière est apparente.

Les analyses théoriques sont conformes aux résultats des tests expérimentaux effectués.

En regard de ces résultats, on peut donc donner la valve de 1 mm comme la plus appropriée pour la réalisation d'une valve de prothèse de larynx.

Le matériau siliconé utilisé lors des essais est biocompatible.

### Exemple 3 : PROTHÈSE DE LARYNX MUNIE D'UN DISPOSITIF À CLAPETS EQUIPÉ D'UN DISPOSITIF D'ASSITANCE MÉCANIQUE

Ce mode de réalisation consiste en un dispositif à clapets selon l'invention dans lequel est ajouté un dispositif aimanté pour augmenter la résistance à la pression dudit système à clapets. Ce système à clapets est placé sur une prothèse de larynx munie d'une bague en titane massif qui viendra renforcer la prothèse dans sa partie distale, au niveau interne.

### A. Dispositif aimanté : un seul aimant

Le dispositif aimanté est constitué d'un aimant Néo-Delta Néodyme-fer-bore (NdFeB) de forme parallélépipédique et de dimensions 2mm x 2mm x 1mm (référence NE22, commercialisé par Binder magnetic®). Cet aimant présente l'avantage d'être très puissant pour une petite taille. Il est aussi rendu parfaitement biocompatible par un traitement anticorrosion.

L'aimant est positionné sur le premier clapet en silicone, à l'opposé de la région charnière.

L'armature porteuse est renforcée au niveau de sa surface interne par une bague en titane massif. Cette bague va porter un élément métallique susceptible d'être aimanté et positionné de façon à pouvoir entrer en contact avec l'aimant quand le premier clapet est en position fermée.

### B. Dispositif aimanté : deux aimants

Le dispositif aimanté est constitué de deux aimants Néo-Delta Néodyme-fer-bore (NdFeB) de forme parallélépipédique et de dimensions 2mm x 2mm x 1mm (référence NE22, commercialisé par Binder magnetic®). Ces aimants sont disposés sur le premier clapet en silicone de part et d'autre d'une verticale joignant la région charnière à son opposée. Ces aimants peuvent être positionnés à des distances variables de cette verticale.

La bague de renfort est munie de deux éléments métalliques susceptibles d'être aimantés et positionnés de façon à pouvoir entrer en contact avec les aimants quand le premier clapet est en position fermée.

### Exemple 4 : PROTHÈSE DE LARYNX MUNIE D'UN DISPOSITIF À CLAPETS EQUIPÉ D'UN DISPOSITIF D'ASSITANCE MÉCANIQUE

Ce mode de réalisation consiste en un dispositif à clapets selon l'invention dans lequel est ajouté un dispositif aimanté pour augmenter la résistance à la pression dudit système à clapets. Ce système à clapets est placé sur une prothèse de larynx munie d'une bague en titane massif qui viendra renforcer la prothèse dans sa partie distale, au niveau interne.

### A. Dispositif aimanté : un seul aimant

Le dispositif aimanté est constitué d'un aimant Néo-Delta Néodyme-fer-bore (NdFeB) de forme parallélépipédique et de dimensions 2mm x 2mm x 1mm (référence NE22, commercialisé par Binder magnetic®). Cet aimant présente l'avantage d'être très puissant pour une petite taille. Il est aussi rendu parfaitement biocompatible par un traitement anticorrosion.

L'aimant est positionné sur l'armature porteuse à l'opposé de la région charnière des clapets.

Le premier clapet est muni d'un élément métallique susceptible d'être aimanté, situé de façon à pouvoir entrer en contact avec l'aimant lorsque le premier clapet est en position fermée.

### B. Dispositif aimanté : trois aimants

Le dispositif aimanté est constitué de trois aimants Néo-Delta Néodyme-fer-bore (NdFeB) de forme parallélépipédique et de dimensions 2mm x 2mm x 1mm (référence NE22, commercialisé par Binder magnetic®). Ces aimants sont disposés sur la bague de renfort de l'armature porteuse et positionnés en trois points différents :
- à l'opposé de la région charnière
- de part et d'autre d'une verticale joignant la région charnière à son opposée ; ces deux aimants peuvent être positionnés à des distances variables de cette verticale. Le premier clapet est muni de trois éléments métalliques susceptibles d'être aimantés et disposés de façon à pouvoir entrer en contact avec les aimants quand le premier clapet est en position fermée.

De manière générale, la présente description a pour but d'illustrer l'invention de la manière la plus claire possible et toute modification de réalisation doit être considérée comme équivalente et, de ce fait, être couverte par les revendications ci-après qui définissent l'étendue de la protection souhaitée.

## Revendications

1. Dispositif à clapets (1) destiné à être implanté au sein d'un larynx dysfonctionnel ou d'une prothèse artificielle de larynx, ledit dispositif comportant une portion distale formant armature porteuse annulaire (3, 3', 3") et une portion centrale formant obturateur (5, 5') destiné à permettre le passage de l'air et à empêcher de manière hermétique le passage de tout autre élément, **caractérisé en ce que** ledit obturateur (5, 5') comprend i) une partie périphérique formant un premier clapet (7, 7', 7") solidaire de l'armature porteuse annulaire (3, 3', 3") au niveau d'une première région charnière (2, 2"), ledit premier clapet (7, 7', 7") étant capable de s'abaisser sous l'effet de la dépression résultant de l'inspiration du patient et ii) une partie centrale formant un second clapet (9, 9', 9") solidaire du premier clapet au niveau d'une seconde région charnière (4, 4"), ledit second clapet (9, 9', 9") étant capable non seulement de se soulever sous l'effet de la surpression exercée par l'air expirée par le patient mais également de coopérer avec le premier clapet (7, 7', 7") pour s'abaisser suite à l'inspiration du patient, lesdits premier et second clapets (7, 7', 7'' ; 9, 9', 9") coopérant entre eux de manière totalement hermétique.

2. Dispositif selon la revendication 1, **caractérisé en ce que** l'armature porteuse annulaire (3, 3', 3") comprend au moins un élément de butée (11, 11') empêchant le soulèvement du premier clapet (7, 7', 7").

3. Dispositif selon la revendication 2, **caractérisé en ce que** ledit élément de butée (11, 11') consiste **en ce que** la circonférence externe du premier clapet (7, 7', 7") de l'obturateur (5) soit découpée en forme de biseau « vers le bas » et **en ce que** la circonférence interne de l'armature porteuse annulaire (3, 3', 3") se trouvant en vis-à-vis soit également découpée en biseau « inversé vers le haut », les deux découpes en biseau coopérant entre elles de manière à bloquer le soulèvement du premier clapet (7, 7', 7").

4. Dispositif selon l'une quelconque des revendications précédentes, **caractérisé en ce que** les premier et second clapets (7, 7', 7" ; 9, 9', 9") comprennent un moyen de coopération de manière à permettre le soulèvement uniquement du second clapet (9, 9', 9") et l'abaissement simultané des premier et second clapets (7, 7', 7" ; 9, 9', 9").

5. Dispositif selon la revendication 4, **caractérisé en ce que** ledit moyen de coopération consiste **en ce que** la circonférence interne du premier clapet (7, 7', 7") soit découpée en biseau « vers le bas » et **en ce que** la circonférence externe du second clapet (9, 9', 9") soit découpée en biseau « inversé vers le haut », les deux découpes en biseau coopérant entre elles.

6. Dispositif selon l'une quelconque des revendications précédentes, **caractérisé en ce que** lesdites première et seconde régions charnières (2, 2" ; 4, 4") présentent une épaisseur comprise entre 0,5 et 3,5 mm.

7. Dispositif selon la revendication 6, **caractérisé en ce que** lesdits premier et second clapets (7, 7', 7" ; 9, 9', 9") présentent une épaisseur comprise entre 0,5 et 3,5 mm.

8. Dispositif selon la revendication 6 ou 7, **caractérisé en ce que** lesdits premier et second clapets (7, 7', 7"; 9, 9', 9") ainsi que lesdites première et seconde régions charnières (2, 2"; 4, 4") présentent la même épaisseur.

9. Dispositif selon l'une quelconque des revendications précédentes, **caractérisé en ce qu'**il est constitué intégralement d'un matériau semi-rigide.

10. Dispositif selon la revendication 9, **caractérisé en ce que** ledit matériau semi-rigide consiste en le silicone.

11. Dispositif selon l'une quelconque des revendications 9 ou 10, **caractérisé en ce que** l'armature porteuse annulaire (3, 3', 3") est renforcée par une ossature en titane, préférentiellement en titane poreux.

12. Dispositif selon l'une quelconque des revendications précédentes **caractérisé en ce que** l'armature porteuse annulaire (3, 3', 3") présente, au niveau de sa circonférence externe, un moyen formant collerette de fixation amovible sur la prothèse de larynx.

13. Dispositif selon la revendication 11, **caractérisé en ce que** ladite collerette de fixation consiste en un système à baïonnette, un système en anneaux enclipsables ou encore en un système à vis.

14. Dispositif selon l'une quelconque des revendications précédentes, **caractérisé en ce que** l'armature porteuse annulaire (3, 3', 3") et/ou le premier clapet (7, 7', 7") est(sont) muni(s) d'un dispositif d'assistance pouvant être mécanique, électrique ou électronique.

15. Dispositif selon la revendication 14, **caractérisé en ce que** ledit dispositif d'assistance consiste en un élément métallique disposé au niveau de l'armature porteuse annulaire (3, 3', 3") venant en contact avec un élément aimanté disposé au niveau du premier clapet (7, 7', 7").

16. Dispositif selon la revendication 14, **caractérisé en ce que** ledit dispositif d'assistance consiste en un élément métallique disposé au niveau du premier clapet (7, 7', 7") venant en contact avec un élément aimanté disposé au niveau de l'armature porteuse annulaire (3, 3', 3").

17. Prothèse artificielle de larynx comprenant, au niveau de sa portion supérieure, un dispositif selon l'une quelconque des revendications 1 à 16.

18. Prothèse artificielle selon la revendication 17 **caractérisée en ce que** ledit dispositif selon l'invention est disposé perpendiculairement à l'axe longitudinal de la prothèse.

19. Prothèse artificielle selon la revendication 17, **caractérisée en ce que** ledit dispositif selon l'invention est disposé incliné par rapport à l'axe longitudinal de la prothèse selon un angle compris entre 0 et 60 degrés.

## Claims

1. Valve device (1) intended to be implanted in a dysfunctional larynx or in an artificial prosthetic larynx, said device comprising a distal portion which forms an annular support structure (3, 3', 3") and a central portion which forms an obturator (5, 5') intended to allow the passage of air and to prevent, in a hermetic manner, the passage of any other element, **characterised in that** said obturator (5, 5') comprises i) a peripheral portion forming a first valve (7, 7', 7") which is integral with the annular support structure (3, 3', 3") at a first hinge region (2, 2"), said first valve (7, 7', 7") being capable of descending under the effect of the negative pressure resulting from the patient inhaling, and ii) a central portion forming a second valve (9, 9', 9") which is integral with the first valve at a second hinge region (4, 4"), said second valve (9, 9', 9") being capable not only of lifting under the effect of the overpressure exerted by the air exhaled by the patient but also of cooperating with the first valve (7, 7', 7") so as to descend after the patient inhales, said first and second valves (7, '7, 7"; 9, 9', 9") cooperating with each other in a fully hermetic manner.

2. Device according to claim 1, **characterised in that** the annular support structure (3, 3', 3") comprises at least one stop element (11, 11') which prevents the first valve (7, 7', 7") from lifting.

3. Device according to claim 2, **characterised in that** said stop element (11, 11') consists **in that** the external circumference of the first valve (7, 7', 7") of the obturator (5) is cut in the shape of a "downward" bevel and **in that** the internal circumference of the opposing annular support structure (3, 3', 3") is also cut to form an "upwardly inverted" bevel, the two bevel cuts cooperating with each other so as to block the lifting of the first valve (7, 7', 7").

4. Device according to any of the preceding claims, **characterised in that** the first and second valves (7, 7', 7"; 9, 9', 9") comprise a cooperation means so as to allow only the second valve (9, 9', 9") to lift and the first and second valves (7, 7', 7"; 9, 9', 9") to descend simultaneously.

5. Device according to claim 4, **characterised in that** said cooperation means consists **in that** the internal circumference of the first valve (7, 7', 7") is cut to form a "downward" bevel and **in that** the external circumference of the second valve (9, 9', 9") is cut to form an "upwardly inserted" bevel, the two bevel cuts cooperating with each other.

6. Device according to any of the preceding claims, **characterised in that** the thickness of said first and second hinge regions (2, 2"; 4, 4") is between 0.5 and 3.5 mm.

7. Device according to claim 6, **characterised in that** the thickness of said first and second valves (7, 7', 7"; 9, 9', 9") is between 0.5 and 3.5 mm.

8. Device according to either claim 6 or claim 7, **characterised in that** said first and second valves (7, 7', 7"; 9, 9', 9") and said first and second hinge regions (2, 2"; 4, 4") are of equal thickness.

9. Device according to any of the preceding claims, **characterised in that** it is made entirely of a semi-rigid material.

10. Device according to claim 9, **characterised in that** said semi-rigid material consists of silicone.

11. Device according to either claim 9 or claim 10, **characterised in that** the annular support structure (3, 3', 3") is reinforces by a titanium framework, preferably made of porous titanium.

12. Device according to any of the preceding claims, **characterised in that**, at the external circumference thereof, the annular support structure (3, 3', 3") has a means forming a removable fixing flange on the larynx prosthesis.

13. Device according to claim 11, **characterised in that** said fixing flange consists of a bayonet system, a system made of clip-on rings or a screw system.

14. Device according to any of the preceding claims, **characterised in that** the annular support structure (3, 3', 3'') and/or the first valve (7, 7', 7") is/are equipped with an assistance device, which may be mechanical, electric or electronic.

15. Device according to claim 14, **characterised in that** said assistance device consists of a metal element, arranged at the level of the annular support structure (3, 3', 3"), which comes into contact with a magnetic element arranged at the level of the first valve (7, 7', 7").

16. Device according to claim 14, **characterised in that** said assistance device consists of a metal element, arranged at the level of the first valve (7, 7', 7"), which comes intro contact with a magnetic element arranged at the level of the annular support structure (3, 3', 3").

17. Artificial larynx prosthesis comprising, at the level of the upper portion thereof, a device according to any of claims 1 to 16.

18. Artificial prosthesis according to claim 17, **characterised in that** said device according to the invention is arranged perpendicular to the longitudinal axis of the prosthesis.

19. Artificial prosthesis according to claim 17, **characterised in that** said device according to the invention is arranged so as to be inclined at an angle between 0 and 60 degrees with respect to the longitudinal axis of the prosthesis.

## Patentansprüche

1. Ventilvorrichtung (1) zur Implantation in einen dysfunktionellen Larynx oder in eine künstliche Larynxprothese, wobei die Vorrichtung einen distalen Anschnitt aufweist, der eine ringförmige Trägerstruktur (3, 3', 3") bildet, und einen mittigen Anschnitt, der einen Verschluss (5, 5') bildet und dafür bestimmt ist, Luft hindurchzulassen und hermetisch das Passieren jedes anderen Elements zu verhindern, **dadurch gekennzeichnet, dass** der Verschluss (5, 5') i) einen Randabschnitt umfasst, der ein erstes Ventil (7, 7', 7") bildet, das mit der ringförmigen Trägerstruktur (3, 3', 3") im Bereich eines ersten Gelenkbereich (2, 2") fest verbunden ist, wobei sich das erste Ventil (7, 7', 7") unter der Einwirkung des Unterdrucks aufgrund des Einatmens des Patienten senken kinn, und zur ii) einen mittigen Abschnitt, der ein zweites Ventil (9, 9', 9") bildet, das mit dem ersten Ventil im Bereich eines zweiten Gelenkbereich (4, 4") fest verbunden ist, wobei das zweite Ventil (9, 9', 9") nicht nur in der Lage ist, sich unter der Einwirkung des Überdrucks, der von der Luft ausgeübt wird, die der Patient ausatmet, zu heben, sondern auch mit dem ersten Ventil (7, 7', 7") zusammenzuwirken, um sich infolge des Einatmens des Patienten zu senken, wobei das erste und zweite Ventil (7, 7', 7"; 9, 9', 9") komplett hermetisch miteinander zusammenwirken.

2. Vorrichtung nach Anspruch 1, **dadurch gekennzeichnet, dass** die ringförmige Trägerstruktur (3, 3', 3") mindestens ein Anschlagelement (11, 11') umfasst, das das Anheben des ersten Ventils (7, 7', 7") verhindert.

3. Vorrichtung nach Anspruch 2, **dadurch gekennzeichnet, dass** das Anschlagelement (11, 11') darin besteht, dass der Außenumfang des ersten Ventils (7, 7', 7") des Verschlusses (5) in Form einer Abschrägung "nach lunten" ausgeschnitten ist und dass der Innenumfang der ringförmigen Trägerstruktur (3, 3', 3"), die gegenüberliegt, ebenfalls als "nach oben umgekehrte" Abschrägung ausgeschnitten ist, wobei die beiden Ausschnitte in Form von Anschrägungen miteinander zusammenwirken, um das Anheben des ersten Ventils (7, 7', 7") zu blockierten.

4. Vorrichtung nach einem der vorhergehenden Ansprüche, **dadurch gekennzeichnet, dass** das erste und zweite Ventils (7, 7', 7"; 9, 9', 9") ein Zusammenwirkungsmittel umfassen, um das Anheben ausschließlich des zweiten Ventils (9, 9', 9") und das gleichzeitige Absenken des ersten und zweiten Ventils (7, 7', 7"; 9, 9', 9") zu ermöglichen.

5. Vorrichtung nach Anspruch 4, **dadurch gekennzeichnet, dass** das Zusammenwirkungsmittel darin besteht, dass der Innenumfang des ersten Ventils (7, 7', 7") als Abschrägung "nach lunten" ausgeschnitten ist und dass der Außenumfang des zweiten Ventils (9, 9', 9") als "nach oben umgekehrte" Abschrägung ausgeschnitten ist, wobei die beiden Ausschnitte in Form von Anschrägungen miteinander zusammenwirken.

6. Vorrichtung nach einem der vorhergehenden Ansprüche, **dadurch gekennzeichnet, dass** der erste und zweite Gelenkbereich (2, 2"; 4, 4") eine Dicke zwischen 0,5 und 3,5 mm aufweisen.

7. Vorrichtung nach Anspruch 6, **dadurch gekennzeichnet, dass** das erste und zweite Ventil (7, 7', 7"; 9, 9', 9") eine Dicke zwischen 0,5 und 3,5 mm aufweisen.

8. Vorrichtung nach Anspruch 6 oder 7, dadurch gekenntzeichnet, dass das erste und zweite Ventil (7, 7', 7"; 9, 9', 9") sowie der erste und zweite Gelenkbereich (2, 2"; 4, 4") dieselbe Dicke aufweisen.

9. Vorrichtung nach einem der vorhergehenden Ansprüche, **dadurch gekennzeichnet, dass** sie vollständig aus einem halbstarren Material gebildet ist.

10. Vorrichtung nach Anspruch 9, **dadurch gekennzeichnet, dass** das halbstarre Material aus Silikon besteht.

11. Vorrichtung nach einem der Ansprüche 9 oder 10, dadurch gekenntzeichnet, dass die ringförmigen Trägerstruktur (3, 3', 3") mit einer Konstruktion aus Titan, vorzuasweise aus porösem Titan, verstärkt ist.

12. Vorrichtung nach einem der vorhergehenden Ansprüche, **dadurch gekennzeichnet, dass** die ringförmigen Trägerstruktur (3, 3', 3") im Bereich ihres Außenumfangs ein Mittel aufweist, das einen abnehmbaren Befestigungsbund an der Larynxprothese bildet.

13. Vorrichtung nach Anspruch 11, dadurch gekenntzeichnet, dass der Befestigungsbund aus einem Bajonettsystem, einem System mit anklemmbaren Ringen oder auch aus einem Schraubsystem besteht.

14. Vorrichtung nach einem der vorhergehenden Ansprüche, **dadurch gekennzeichnet, dass** die ringförmige Trägerstruktur 3, 3', 3") und/oder das erste Ventil (7, 7', 7") mit einer Hilfsvorrichtung, die mechanisch, elektrisch oder elektronisch sein kann, versehen ist bzw. sind.

15. Vorrichtung nach Anspruch 14, dadurch gekenntzeichnet, dass die Hilfsvorrichtung aus einem metallischen Element, besteht, das im Bereich der ringförmigen Trägerstruktur (3, 3', 3") angeordnet ist und mit einem magnetische Element, das im Bereich des ersten Ventils (7, 7', 7") angeordnet ist, in Berührung kommt.

16. Vorrichtung nach Anspruch 14, **dadurch gekennzeichnet, dass** die Hilfsvorrichtung aus einem metallischen Element, besteht, das im Bereich des ersten Ventils (7, 7', 7") angeordnet ist und mit einem magnetischen Element, das im Bereich der Trägerstruktur (3, 3', 3") angeordnet ist, in Berührung kommt.

17. Künstliche Larynxprothese, die im Bereich ihres oberen Abschnitts eine Vorrichtung nach einem der Ansprüche 1 bis 16 umfasst.

18. Künstliche Prothese nach Anspruch 17, **dadurch gekennzeichnet, dass** die erfindungsgemäße Vorrichtung senkrecht zur Längsachse der Prothese angeordnet ist.

19. Künstliche Prothese nach Anspruch 17, **dadurch gekennzeichnet, dass** die erfindungsgemäße Vorrichtung bezogen auf die Längsachse der Prothese unter einem Winkel zwischen 0 und 60 Grad geneigt angeordnet ist.
